# EUROPEAN PATENT SPECIFICATION

(11) **EP 1 242 028 B1**
(45) Date of publication and mention of the grant of the patent: **18.05.2005**
(21) Application number: 00978176.6
(22) Date of filing: 17.11.2000
(51) Int. Cl.: A61F 13/472

(54) **ABSORBENT ARTICLE WITH RAISED REAR PORTION FOR USE IN A THONG GARMENT**
ABSORBIERENDER ARTIKEL MIT ERHÖTER HINTERER REGION ZUR BENUTZUNG IN EINEM RIEMENHÖSCHEN
ARTICLE ABSORBANT POURVU D'UNE PARTIE ARRIERE RELEVEE UTILISABLE AVEC DES MINI-SLIPS

(30) Priority: 18.11.1999 SE 9904199
(43) Date of publication of application: 25.09.2002
(73) Proprietor: SCA Hygiene Products AB, 405 03 Göteborg (SE)
(72) Inventor: PERSSON, Charlotte, S-413 19 Göteborg (SE)
(74) Representative: Romare, Laila Anette
(86) International application number: PCT/SE2000/002257
(87) International publication number: WO 2001/035887

(56) References cited:
- WO-A1-97/39713
- WO-A1-98/22062
- WO-A1-98/51249
- FR-A3- 2 763 839

## Description

### TECHNICAL FIELD

The invention relates to an absorbent product intended for women for use in a thong, the product being essentially elongate in shape with a longitudinal direction, a transverse direction and a thickness direction, an upper side, a lower side and two longitudinal side edges, and comprising a liquid-permeable surface layer, a liquidtight surface layer, and an absorption body arranged between the two surface layers, the product also having a front portion arranged so as to face forwards on a wearer and a rear portion arranged so as to face backwards on a wearer when the product is in use, the upper side of the product having a longitudinal raised portion with a length of 30-100 mm and a maximum width of 25 mm.

### BACKGROUND ART

Absorbent products such as sanitary towels and panty liners are intended to be worn in close contact with the body of the wearer. In this connection, such an absorbent product is usually arranged inside the briefs of the wearer and is kept in contact with the body during use by pressure from the briefs. However, it has become much more common for women to wear what are known as thongs, that is to say briefs with an extremely small rear portion. In this connection, a problem is that the sanitary towels and panty liners which have been available until now are designed to fit in conventional briefs. When used together with thongs, a number of problems therefore arise. One practical problem is that it is virtually impossible to fasten a conventional sanitary towel or a panty liner in a thong in such a manner that the towel or the panty liner sits correctly in relation to the body of the wearer and is moreover held in position throughout use. For example, the risk that the absorbent product moves laterally is very great, which means that the risk of leakage is imminent. Another major problem is linked to the fact that thongs are often worn for aesthetic reasons because they are virtually invisible even under clinging clothes and do not give rise to unsightly edge lines or creases in the clothes. With conventional absorbent products, a large part of the desired aesthetic effect of wearing a thong is of course lost. In order to solve this problem, designing an absorbent product adapted to the shape of a thong has been proposed. Such products are described in WO 97/39713 and SE 9803981-1 and further in WO 98151249. Adaptation of the shape of the absorbent product means, however, that the product must be designed with a very narrow rear portion. A difficulty in this connection is to produce a product with sufficiently great absorption capacity. Moreover, it is necessary that the product can be positioned in such a manner in relation to the body of the wearer that no liquid runs out past the edges of the product and that the absorption capacity of the product can be put to maximum use.

By means of the present invention, an absorbent product has been produced, which is intended for use together with a thong, which product essentially eliminates the problems mentioned above.

A product made according to the invention is characterized mainly in that the greatest width of the front portion (7) is at least 55 mm, and in that the maximum total length of the product is 260 mm, the raised portion having a rear part and a front part, the rear part having greater rigidity in the thickness direction of the product than the front part.

According to one embodiment of the invention, the raised portion is positioned mainly on the rear portion of the product and extends from the rear portion in the direction towards the front edge of the product. It has proved suitable to design the raised portion with an essentially triangular cross section, that is to say with a wider base and a narrower top.

By virtue of the fact that an absorbent product according to the invention is designed so as to fit in a thong and has a very narrow rear portion, the available liquid-receiving surface area on the product is smaller than for conventional absorbent products. It is therefore suitable if the raised portion comprises superabsorbent material which, in relation to its volume, can absorb great quantities of bodily fluid. It is usually estimated that a panty liner should have an absorption capacity of roughly 3-5 ml and a sanitary towel should be capable of absorbing roughly 12-15 ml. For products intended for night use, for example, or for incontinence pads, an even greater absorption capacity may be desirable.

According to the invention, the raised portion can comprise odour-inhibiting means.

It is also suitable if the product has an anatomically adapted shape. The side edges of the product are therefore advantageously curved. The transition from the width of the front portion to the width of the rear portion can then take place within 5-20 mm of the length of the product. This is advantageous because such a design of the product contributes to facilitating correct positioning of the product in relation to the body of the wearer, and results in the product staying in position better during use by preventing the product sliding backwards.

In order further to increase the inconspicuousness when the product is worn in a thong, the product can be designed with a maximum total length of 230 mm, and preferably at least 140 mm.

The product can be provided with a fastening means for fastening the product in a thong. Suitable fastening means are, for example, adhesive, touch and close fasteners, friction coatings or the like. The fastening means can also comprise side flaps or wings which are suitably arranged on the rear portion of the product. Such side flaps are folded around the crotch of the briefs and fastened to the briefs or to one another.

The height of the raised portion above the surface of the absorbent product should be adapted in such a manner that good contact between the body of the wearer and the raised portion is ensured during use. As the raised portion, at least within a rear portion, is advantageously so hard or rigid that it essentially maintains its shape during use, the raised portion should not be so high that it presses against the body of the wearer during use and thus causes discomfort. It is also necessary to ensure that the raised portion does not chafe the sensitive soft parts in the crotch of the wearer. It has been found that a raised portion which, in its highest portion, projects upwards at least 5 mm but not more than 20 mm from the surface of the product satisfies the requirements for both good bodily contact and a high degree of wearer comfort.

A raised portion according to the invention is relatively narrow, suitably at most 25 mm, and preferably between 12 mm and 16 mm, at the base. At the top, the raised portion is suitably between 3 mm and 10 mm, and preferably between 4 mm and 6 mm, wide. The raised portion has an essentially triangular cross section and is therefore wider at the base than at the top. By virtue of the fact that the raised portion is relatively narrow, it can project inwards slightly between the labia of the wearer without discomfort for the wearer. In this connection, it is an advantage if the raised portion parts the labia slightly because liquid transfer from the wearer to the product is in this way facilitated.

In the rear part of the raised portion, it is advantageous if the raised portion has such a shape that it bears in close contact against the body of the wearer, in the area behind the vaginal orifice. In this way, bodily fluid is prevented from running backwards in the cleft between the buttocks of the wearer and leaking out of the product. Such backward leakage is especially troublesome when the wearer is lying down. In a corresponding manner, the front part of the raised portion should follow the shape of the body of the wearer in the area in front of the vaginal orifice. As mentioned previously, the front part of the raised portion is suitably softer in the thickness direction of the product than the rear part of the raised portion.

It is important that the bodily fluid discharged is caught and absorbed immediately into the raised portion. In this way, liquid is prevented from collecting between the body of the wearer and the raised portion. This is important because, when the wearer rises or moves in another manner, there is a risk of a gap occurring in the front part of the raised portion, between the raised portion and the body of the wearer. If a relatively large quantity of unabsorbed liquid has become trapped between the body of the wearer and the surface of the product, such liquid can then flow out through the gap. Such sudden liquid flows are particularly undesirable, on the one hand because they are an unpleasant experience for the wearer and on the other hand because they increase the risk of leakage considerably.

In order to follow the shape of the anatomy of the wearer, the raised portion should be highest in that part of the product which is intended to bear against the vaginal orifice of the wearer. From the highest part, the height should decrease gradually in the direction towards the end portions of the product. In this connection, the raised portion should extend backwards from the highest portion for between 5 mm and 40 mm, preferably between 10 mm and 35 mm. In front of the highest portion, the raised portion should have a length of between 50 mm and 85 mm, preferably between 55 mm and 80 mm.

### DESCRIPTION OF DRAWINGS

The invention will be described in greater detail below with reference to the exemplary embodiments shown in the appended drawings, in which:
- Figure 1: shows a sanitary towel according to an embodiment of the invention, and
- Figure 2: shows a section along the line II-II through the sanitary towel in Figure 1.

### MODE(S) FOR CARRYING OUT THE INVENTION

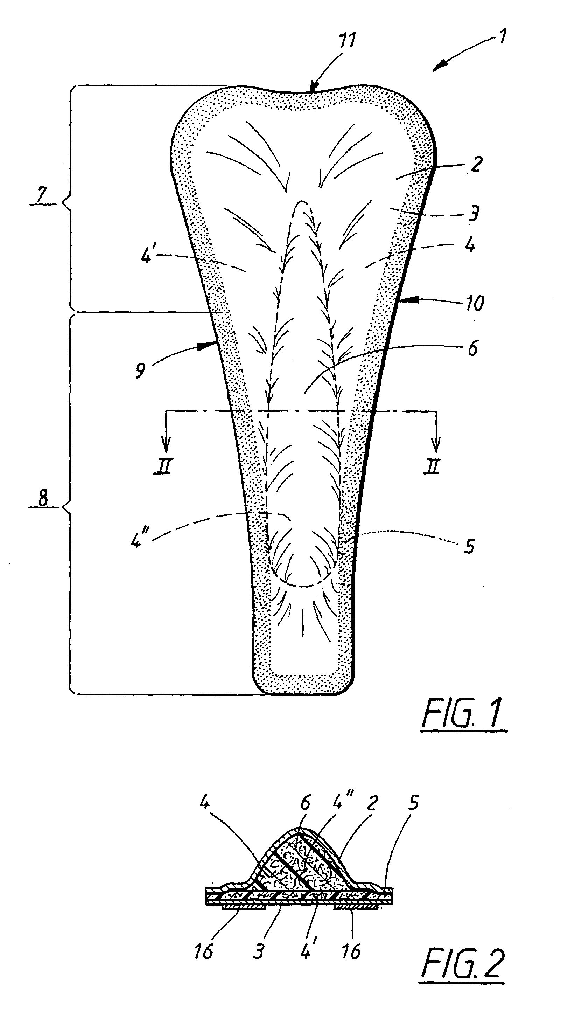

The sanitary towel 1 shown in Figures 1 and 2 comprises a liquid-permeable surface layer 2 arranged on that side of the sanitary towel which is intended to face a wearer during use, a liquidtight surface layer 3 arranged on that side of the sanitary towel which is intended to face away from the wearer during use, and an absorption body 4 enclosed between the two surface layers 2, 3. The liquid-permeable surface layer 2 has essentially the same shape and extent in the plane as the absorption body 4 and is joined together with the absorption body 4 and the liquidtight surface layer 3 in a join 5 around the entire periphery of the sanitary towel. Such a compressed join forms a leakage barrier and prevents liquid leaking out of the sanitary towel in the edge portions. For sanitary towels which are intended to absorb relatively great quantities of bodily fluid, it is suitable for the edge join to be very greatly compressed or to contain thermoplastic material which is melted to form a liquidtight barrier. It is also possible to produce a liquid leakage barrier by folding the liquidtight surface layer around the edges or by saturating the edge portions with liquidtight adhesive. According to an alternative embodiment (not shown), the two surface layers 2, 3 can have a slightly greater extent in the plane than the absorption body 4 and be interconnected outside the absorption body, as a result of which a projecting covering edge is formed around the absorption body. The surface layers 2, 3 and the absorption body are interconnected by, for example, gluing, sewing or welding using heat or ultrasound.

The liquid-permeable surface layer 2 can therefore consist of any type of liquid-permeable material suitable for the purpose. Examples of such material are different types of thin nonwoven material, perforated plastic films, net material, liquid-permeable foam material or the like. The liquid-permeable surface layer 2 can be constructed from two or more different materials in order to provide different functions of the surface layer. For example, it is usual to arrange a liquid-transporting layer inside a liquid-admission layer. It is also known to arrange different types of material on different parts of that surface on the sanitary towel which faces the wearer during use. A material with good admission capacity can therefore advantageously be arranged in that portion of the sanitary towel which is expected to be moistened first by the major part of the bodily fluid, while portions of the surface layer which are in the first instance to constitute a contact surface against the body of the wearer are provided with a material which has been optimized with regard to softness and kindness to the skin.

As described in PCT/SE98/00187, the liquid-permeable surface layer 2 can also consist of a first layer of conventional, hydrophobic liquid-permeable material. The first layer is arranged over that surface on the absorption body 4 which is intended to face the wearer during use. Examples of hydrophobic surface materials are perforated plastic films, hydrophobic nonwoven materials, plastic net material or the like. A hydrophobic surface material allows liquid to pass through to the absorption body 4 lying inside. As the absorption body is more hydrophilic than the material in the hydrophobic surface material, the surface material is drained virtually completely of liquid. For this reason, and because the surface material essentially has no absorption capacity, the surface material remains dry even after being moistened. Only a very small quantity of liquid can remain on or in such a hydrophobic surface layer.

During use of the sanitary towel 1, it is arranged in the genital area of the wearer, with a portion located in the region of the vaginal orifice of the wearer. As a result, discharged bodily fluid will meet the sanitary towel 1 within a limited area on the sanitary towel, what is known as the wet area. Within the wet area, the liquid-permeable surface layer 2 therefore suitably has a second, hydrophilic and absorbent layer. Examples of suitable hydrophilic materials are in this connection nonwoven materials comprising rayon, cotton, cellulose fibres or the like.

During use, the hydrophilic absorbent layer can come into contact with the sensitive mucous membranes inside the labia of the wearer. By virtue of the fact that the layer is hydrophilic and absorbent, there is no risk of the mucous membranes drying out because some of the liquid absorbed by the sanitary towel will remain in the hydrophilic absorbent layer. As a result, the mucous membranes are kept moist during use and the risk of chafing and other irritation of the mucous membranes is virtually entirely eliminated. Furthermore, the risk of liquid running on the surface of the product before it is absorbed into the product is very small because the hydrophilic layer catches and absorbs discharged bodily fluid immediately.

In contrast to the mucous membranes, the skin of the wearer in the genital area should be protected against moistening. Within those parts of the surface of the sanitary towel 1 which are in contact with the skin of the wearer during use, the liquid-permeable surface layer 2 is therefore suitably hydrophobic. As a result, a high degree of dryness against the skin is achieved, and irritation of the same is avoided.

It is not necessary either for the invention that the liquid-permeable surface layer 2 consists of a separate material layer, but the surface layer 2 can be a surface on the absorption body 4 of the sanitary towel. In such an embodiment, however, it is particularly suitable to provide the sanitary towel with some form of liquid barrier which prevents liquid from being transported in the absorption material to the very edges of the sanitary towel. Examples of such liquid barriers are compressions, welds, strands of adhesive, folded-over plastic strips or means of rendering materials hydrophobic, such as wax or the like.

The liquidtight surface layer 3 can be made of any liquidtight material suitable for the purpose. Common barrier layers are thin plastic films made of, for example, polyethylene or polypropylene. It is also possible, however, to use impermeable nonwoven materials, impermeable foam materials or the like. Coating with liquidtight materials is another possibility. The liquidtight surface layer can be air-permeable and vapour-permeable but impermeable to liquid. It can also be advantageous to use stretchable or elastically stretchable barrier layers.

The absorption body 4 consists of two parts, a first part 4' which is relatively thin and is in the form of one or more material layers which extend over the entire surface of the sanitary towel, and a second part 4" which forms an absorbent raised portion 6. Suitable absorbent materials for use in the absorption body 4 are, for example, cellulose fluff pulp, absorbent bound fibre layers, tissue layers, absorbent foam, peat or the like. The absorption body can also contain superabsorbent polymers, that is to say polymers with the capacity to absorb several times their own weight of liquid, forming a liquid-containing gel. Superabsorbents are usually in the form of particles, flakes, fibres, granules or the like. The superabsorbent material can be used on its own or together with other absorbent material. As a sanitary towel to be used together with a thong is very narrow, this means that the surface area of the liquid-receiving area is small. For this reason, it may be suitable to arrange absorbent material with a great absorption capacity within the area which receives and absorbs liquid. It is therefore particularly suitable for the raised portion 6 to contain superabsorbent material. Superabsorbents are also capable of chemically binding the absorbed liquid, which means that the risk of absorbed liquid leaking back out of the sanitary towel is minimal. This is also particularly advantageous of course when the size of the absorption body is limited.

The sanitary towel 1 is designed with a relatively wide front portion 7 and a considerably narrower rear portion 8. The sanitary towel 1 also has two side edges 9, 10, the main extent of which is in the longitudinal direction of the sanitary towel, and an essentially transverse front edge 11.

As mentioned above, the sanitary towel has a raised portion 6 arranged on that surface of the sanitary towel which is intended to face the wearer during use. The raised portion 6 is elongate and positioned centrally along a longitudinal centre line through the sanitary towel. The raised portion 6 suitably has a triangular cross-sectional shape with a wider and higher part at the rear and a narrower and lower part at the front. It is also advantageous if the rear part of the raised portion 6 is stiffer in the thickness direction of the sanitary towel than the front part of the raised portion 6. It has been found suitable for the raised portion to be roughly 30-100 mm long and to have a width not exceeding 25 mm. A particularly suitable raised portion with great dimensional stability is described in international patent application WO 98/22062.

The raised portion 6 serves a number of purposes. On the one hand, it constitutes a positioning means by virtue of the fact that the wearer can position the sanitary towel correctly in relation to the body on the basis of the fact that the raised portion 6 will be positioned in the region of the vaginal orifice. On the other hand, the raised portion 6 ensures that a sufficient quantity of absorption material is available to absorb discharged bodily fluid as soon as it leaves the body of the wearer. By virtue of the fact that the raised portion 6 is narrow, it will be introduced slightly between the labia of the wearer during use, as a result of which it counteracts lateral movement of the rear portion 8 of the sanitary towel. The raised portion therefore contributes to holding the sanitary towel 1 in position during use.

As can be seen from Figure 2, the raised portion 6 is shaped by virtue of the fact that absorption material has been accumulated in a ridge-shaped strand along the longitudinal centre line of the sanitary towel. It is alternatively possible to make the raised portion 6 from a combination of absorbent material and shaping components such as stiffening members or the like. In the event that only a very small absorption capacity is required and the raised portion 6 is intended primarily as a positioning means and to prevent lateral displacement of the sanitary towel, the raised portion 6 can be entirely non-absorbent. Such an embodiment is suitable for, for example, panty liners where a very thin absorption layer or the absorption capacity provided by an absorbent surface layer 2 alone may be sufficient.

As can be seen in Figure 2, a fastening means 16 in the form of two strands of self-adhesive glue extending along the side edges 9, 10 is arranged on the outside of the liquidtight covering layer 3. During use of the sanitary towel 1, it is arranged inside a thong and is fastened in the briefs by means of the fastening means 16. Before use, the fastening means 16 is protected in a conventional manner, for example by being covered by a protective layer of paper or plastic treated with silicone or stamped so as to be easily detachable from the adhesive when the sanitary towel is to be used. The adhesive can of course be arranged in any pattern suitable for the purpose. It is also possible to use other types of fastening means, such as friction coatings, press-studs, clips, fastening flaps or the like. Another alternative is fastening adhesive which is attached to the body of the wearer.

Although the invention is described above in connection with a sanitary towel, it can of course also be applied to other types of absorbent product intended to be used by women and to be worn inside a thong.

## Claims

1. An absorbent product being elongate in shape with a longitudinal direction, a transverse direction and a thickness direction, an upper side, a lower side and two longitudinal side edges, and comprising a liquid-permeable surface layer (2), a liquidtight surface layer (3), and an absorption body (4) arranged between the two surface layers (2, 3), the product also having a front portion (7) arranged so as to face forwards on a wearer when the product is in use and a rear portion (8) arranged so as to face backwards on a wearer when the product is in use, the upper side of the product having a longitudinal raised portion with a length of 30-100 mm and a maximum width of 25 mm, **characterized in that** the rear portion (8) has a smallest width of less than 30 mm, the greatest width of the front portion (7) is at least 55 mm, and **in that** the maximum total length of the product is 260 mm, the raised portion having a rear part and a front part, the rear part having greater rigidity in the thickness direction of the product than the front part.

2. Absorbent product according to Claim 1, the raised portion being positioned mainly on the rear portion (8) of the product.

3. Absorbent product according to Claim 1 or 2, the raised portion (6) having a triangular cross section.

4. Absorbent product according to any one of the preceding claims, the raised portion comprising superabsorbent material.

5. Absorbent product according to any one of the preceding claims, the raised portion comprising odour-inhibiting means.

6. Absorbent product according to any one of the preceding claims, the side edges of the product being curved.

7. Absorbent product according to any one of the preceding claims, the transition from the width of the front portion (7) to the width of the rear portion (8) taking place within 5-20 mm of the length of the product.

8. Absorbent product according to any one of the preceding claims, the product having a maximum total length of 155 mm.

9. Absorbent product according to any one of the preceding claims, the 'product being provided with a fastening means (16) for fastening the product in a thong.

10. Absorbent product according to Claim 9, the fastening means comprising side flaps which are arranged on the rear portion of the product.

11. Absorbent product according to any one of the preceding claims, the maximum total length of the product being at least 140 mm.

12. Absorbent product, according to any one of the preceding claims, the maximum total length of the product being 230 mm.

13. Absorbent product, according to any one of the preceding claims, the maximum width is between 12 mm and 16 mm.

## Patentansprüche

1. Absorbierendes Produkt von länglicher Form mit einer Längsrichtung, einer Querrichtung und einer Dickenrichtung, einer oberen Seite, einer unteren Seite und zwei Längsseitenkanten, und umfassend eine flüssigkeitsdurchlässige Oberflächenschicht (2), eine flüssigkeitsundurchlässige Oberflächenschicht (3), und einen zwischen den zwei Oberflächenschichten (2, 3) angeordneten Absorptionskörper (4), wobei das Produkt zudem einen Vorderabschnitt (7) aufweist, der so angeordnet ist, dass er bei einem Träger nach vorne gewandt ist, wenn das Produkt in Gebrauch ist, und einen hinteren Abschnitt (8) aufweist, der so angeordnet ist, dass er bei einem Träger nach hinten gewandt ist, wenn das Produkt in Gebrauch ist, wobei die obere Seite des Produkts einen länglichen erhöhten Abschnitt mit einer Länge von 30 bis 100 mm und einer maximalen Breite von 25 mm aufweist, **dadurch gekennzeichnet, dass** der hintere Abschnitt (8) eine kleinste Breite von weniger als 30 mm aufweist, die größte Breite des Vorderabschnitts (7) mindestens 55 mm beträgt, und dass die maximale totale Länge des Produkts 260 mm beträgt, und wobei der erhöhte Abschnitt einen hinteren Teil und einen vorderen Teil aufweist, wobei der hintere Teil eine größere Steifheit in der Dickenrichtung des Produkts aufweist als der vordere Teil.

2. Absorbierendes Produkt nach Anspruch 1, wobei der erhöhte Abschnitt hauptsächlich auf dem hinteren Abschnitt (8) des Produkts positioniert ist.

3. Absorbierendes Produkt nach Anspruch 1 oder 2, wobei der erhöhte Abschnitt (6) einen dreieckigen Querschnitt aufweist.

4. Absorbierendes Produkt nach einem der vorhergehenden Ansprüche, wobei der erhöhte Abschnitt ein superabsorbierendes Material umfasst.

5. Absorbierendes Produkt nach einem der vorhergehenden Ansprüche, wobei der erhöhte Abschnitt geruchshemmende Mittel umfasst.

6. Absorbierendes Produkt nach einem der vorhergehenden Ansprüche, wobei die Seitenkanten des Produkts gebogen sind.

7. Absorbierendes Produkt nach einem der vorhergehenden Ansprüche, wobei der Übergang von der Breite des Vorderabschnitts (7) zu der Breite des Hinterabschnitts (8) innerhalb von 5 bis 20 mm der Länge des Produkts stattfindet.

8. Absorbierendes Produkt nach einem der vorhergehenden Ansprüche, wobei das Produkt eine maximale totale Länge von 155 mm aufweist.

9. Absorbierendes Produkt nach einem der vorhergehenden Ansprüche, wobei das Produkt mit einer Befestigungsvorrichtung (16) zur Befestigung des Produkts in einem Riemenhöschen versehen ist.

10. Absorbierendes Produkt nach Anspruch 9, wobei die Befestigungsvorrichtung Seitenlaschen umfasst, welche auf dem hinteren Abschnitt des Produkts angeordnet sind.

11. Absorbierendes Produkt nach einem der vorhergehenden Ansprüche, wobei die maximale totale Länge des Produkts mindestens 140 mm beträgt.

12. Absorbierendes Produkt nach einem der vorhergehenden Ansprüche, wobei die maximale totale Länge des Produkts mindestens 230 mm beträgt.

13. Absorbierendes Produkt nach einem der vorhergehenden Ansprüche, wobei die maximale Breite zwischen 12 mm und 16 mm beträgt.

## Revendications

1. Article absorbant de forme allongée ayant une direction longitudinale, une direction transversale et une direction d'épaisseur, un côté supérieur, un côté inférieur et deux bords latéraux longitudinaux, et comprenant une couche de surface perméable (2) aux liquides, une couche de surface imperméable (3) aux liquides et un corps absorbant (4) placé entre les deux couches de surface (2, 3), l'article comportant aussi une partie avant (7) agencée de manière à être tournée vers l'avant sur un utilisateur lorsque l'article est en utilisation et une partie arrière (8) agencée de manière à être tournée vers l'arrière sur un utilisateur lorsque l'article est en utilisation, le côté supérieur de l'article comportant une partie surélevée longitudinale ayant une longueur de 30 à 100 mm et une largeur maximale de 25 mm, **caractérisé en ce que** la plus petite largeur de la partie arrière (8) est inférieure à 30 mm, la plus grande largeur de la partie avant (7) fait au moins 55 mm, et **en ce que** la longueur totale maximale de l'article est de 260 mm, la partie surélevée comportant une partie arrière et une partie avant, la partie arrière ayant une plus grande rigidité dans le sens de l'épaisseur de l'article que la partie avant.

2. Article absorbant selon la revendication 1, dans lequel la partie surélevée est positionnée principalement sur la partie arrière (8) de l'article.

3. Article absorbant selon la revendication 1 ou 2, dans lequel la partie surélevée (6) est de section triangulaire.

4. Article absorbant selon l'une quelconque des revendications précédentes, dans lequel la partie surélevée comprend un matériau superabsorbant.

5. Article absorbant selon l'une quelconque des revendications précédentes, dans lequel la partie surélevée comprend un moyen neutralisant les odeurs.

6. Article absorbant selon l'une quelconque des revendications précédentes, dans lequel les bords latéraux de l'article sont courbés.

7. Article absorbant selon l'une quelconque des revendications précédentes, dans lequel la transition de la largeur de la partie avant (7) à la largeur de la partie arrière (8) a lieu dans une plage de 5 à 20 mm de la longueur de l'article.

8. Article absorbant selon l'une quelconque des revendications précédentes, dans lequel l'article a une longueur totale maximale de 155 mm.

9. Article absorbant selon l'une quelconque des revendications précédentes, dans lequel l'article est muni d'un moyen de fixation (16) pour fixer l'article dans un string.

10. Article absorbant selon la revendication 9, dans lequel le moyen de fixation comprend des rabats latéraux qui sont placés sur la partie arrière de l'article.

11. Article absorbant selon l'une quelconque des revendications précédentes, dans lequel la longueur totale maximale de l'article est supérieure ou égale à 140 mm.

12. Article absorbant selon l'une quelconque des revendications précédentes, dans lequel la longueur totale maximale de l'article est de 230 mm.

13. Article absorbant selon l'une quelconque des revendications précédentes, dans lequel la largeur maximale est comprise entre 12 mm et 16 mm.
